# EUROPÄISCHE PATENTSCHRIFT

(11) **EP 0 789 714 B1**
(45) Veröffentlichungstag und Bekanntmachung des Hinweises auf die Patenterteilung: **13.06.2001**
(21) Anmeldenummer: 95938392.8
(22) Anmeldetag: 02.11.1995
(51) Int. Cl.: C07K 16/00, C07K 16/06, G01N 33/563, G01N 33/68, G01N 33/543

(54) **ANTIKÖRPERKLASSENSPEZIFISCHES ENTSTÖRUNGSREAGENZ**
INTERFERENCE-SUPPRESSING REAGENT SPECIFIC TO CERTAIN CLASSES OF ANTIBODIES
REACTIF ELIMINANT LES INTERFERENCES SPECIFIQUE A DES CLASSES D'ANTICORPS

(30) Priorität: 04.11.1994 DE 4439452
(43) Veröffentlichungstag der Anmeldung: 20.08.1997
(73) Patentinhaber: Roche Diagnostics GmbH, 68305 Mannheim (DE)
(72) Erfinder: SCHLIEPER, Dittmar, D-82393 Iffeldorf (DE); LENZ, Helmut, D-82327 Tutzing (DE); SCHMITT, Urban, D-82386 Oberhausen (DE); KLEMT, Volker, D-82362 Weilheim (DE)
(74) Vertreter: Weiss, Wolfgang, Dipl.-Chem. Dr.
(86) Internationale Anmeldenummer: EP9504307
(87) Internationale Veröffentlichungsnummer: WO9614337

(56) Entgegenhaltungen:
- EP-A- 0 292 810
- PATENT ABSTRACTS OF JAPAN vol. 006 no. 073 (C-101) ,8.Mai 1982 & JP,A,57 009723 (MITSUBISHI CHEM IND LTD) 19.Januar 1982,
- The Journal of Rheumatology 12, 3, 1985, 427 - 431

## Beschreibung

Die Erfindung betrifft eine aus mehreren verschiedenen Antikörpern oder/und Antikörperfragmenten bestehende Zusammensetzung, die als Entstörungsreagenz bei einem immunologischen Verfahren zum klassenspezifischen Nachweis von Antikörpern aus einer oder mehreren der Immunglobulinklassen G, M, A, D und E geeignet ist.

Der Säugetierorganismus enthält verschiedene Klassen von Antikörpern, die von B-Zellen des Immunsystems zur Bekämpfung von Antigenen gebildet werden. Die Antikörpermoleküle bestehen aus einem oder mehreren Sätzen von jeweils vier Polypeptidketten, zwei schweren Ketten und zwei leichten Ketten, die miteinander über Disulfidbrücken verbunden sind.

Antikörper werden im allgemeinen in die Klassen G, M, A, D und E eingeteilt. Diese fünf Immunglobulinklassen unterscheiden sich jeweils in der schweren Kette, die als γ-, µ-, α-, δ- bzw. ∈-Kette bezeichnet wird. Darüber hinaus gibt es bei IgG, IgA und IgM auch Immunglobulinsubklassen.

Antikörper der Klasse IgG bilden in normalem menschlichem Serum 70 bis 75 % des Gesamtimmunglobulins (entsprechend 8 bis 16 mg/ml). Sie werden hauptsächlich als sekundäre Immunantwort des Organismus auf eine Infektion gebildet.

Antikörper der Klasse IgM bilden ca. 10 % des im humanen Serum vorhandenen Immunglobulins und besitzen eine pentamere Struktur. Diese Antikörper treten sehr früh nach einer Infektion auf, so daß ihre Bestimmung zur Früherkennung von Krankheiten bedeutsam ist.

Die Immunglobuline der Klasse IgA bilden ca. 15 bis 20 % des im humanen Serum vorhandenen Immunglobulins und stellen das wichtigste sekretorische Immunglobulin in Speichel, Milch und Sekreten des Urogenitalbereichs dar.

Antikörper der Klasse IgD finden sich auf der Membran zirkulierender B-Zellen und man nimmt an, daß sie bei Autoimmunkrankheiten eine Rolle spielen.

Antikörper der Klasse IgE kommen im Serum nur in sehr geringem Anteil vor, sie spielen aber eine wichtige Rolle bei einer Reihe allergischer Reaktionen, wie etwa Asthma und Heuschnupfen.

Die klassenspezifische Bestimmung von Immunglobulinen, d.h. die selektive Bestimmung von Antikörpern einer oder mehrerer ausgewählter Immunglobulinklassen oder -subklassen, die gegen ein bestimmtes Antigen gerichtet sind, in Gegenwart von Antikörpern anderer Immunglobulinklassen oder -subklassen, die gegen das gleiche Antigen gerichtet sind, ist von besonderer Bedeutung für den Nachweis bestimmter Erkrankungen, z.B. zur Früherkennung von Infektionen, zur Unterscheidung akuter von ausgeheilten Infektionen und zum Treffen präziser prognostischer Aussagen.

Verfahren zur klassenspezifischen Bestimmung von Immunglobulinen sind bekannt. Hierzu kann eine Immunkomponente, die für eine ausgewählte Antikörperklasse spezifisch ist, z.B. ein Antikörper gegen die µ-Kette von humanem IgM, an einen festen Träger gekoppelt werden und der antigenspezifische Immunglobulinanteil durch Reaktion mit einem direkt oder indirekt markierten Antigen nachgewiesen werden. Ein Nachteil bei Verfahren dieses Typs besteht darin, daß nicht-antigenspezifische Immunglobuline der jeweils ausgewählten Immunglobulinklasse mit den antigenspezifischen Molekülen um den Festphasenantikörper konkurrieren. Dadurch kann es in Abhängigkeit von dieser Mengenrelation zu Ergebnisverfälschungen kommen.

Ein weiterer Nachteil der Verfahren des Standes der Technik besteht darin, daß nach Inkubation der Probe mit der Immunkomponente am festen Träger und vor Umsetzung mit der antigenspezifischen Immunkomponente ein Waschungsschritt erforderlich ist. Dies bedeutet eine potentielle Fehlerquelle und eine erhebliche Verlängerung der Testzeit.

Powell et al. (J. Rheumat. 12 (1985), 427-431) offenbaren die Verwendung eines radiomarkierten Anti-Human-IgG-Fd-Antiserums aus Schafen als Nachweisreagenz in einem Test auf IgG-Rheumatoidfaktoren. Eine Antikörper- oder/und Antikörperfragmentzusammensetzung, die spezifisch für den Fd-Abschnitt einer schweren Immunglobulinkette ist, wird jedoch nicht beschrieben. Es finden sich auch nur unvollständige Angaben über die Gewinnung des Fd-Antiserums.

EP-B-0 292 810 offenbart ein Verfahren zur Bestimmung von antigenspezifischen Antikörpern aus einer der Immunglobulinklassen M, A, D und E in einer Probeflüssigkeit, wobei zur Vermeidung von Störungen durch Antikörper der Klasse IgG ein Entstörungsreagenz zugesetzt wird, das aus Anti-Human-IgG, aggregiertem humanem oder tierischem IgG oder einem γ-Fc-Fragment ausgewählt ist. Das Entstörungsreagenz soll die Antigenbindung der in der Probe vorhandenen spezifischen IgG-Antikörper beseitigen und die Aktivität von Rheumafaktoren unterbinden.

Die Entstörungsreagenzien gemäß EP-B-0 292 810 weisen jedoch einige Nachteile auf. So werden unlösliche Immunkomplexe erzeugt, die zur Trübung der Probe und gegebenenfalls zu Störungen bei der Messung führen können. Noch wichtiger ist, daß die offenbarten Reagenzien die Antigenbindungsstelle des IgG nicht maskieren, so daß selbst bei einer Sedimentation noch eine Antigenbindung und somit eine Verfälschung des Tests stattfinden kann. Bei Einsatz von Anti-Human-IgG-Antikörpern muß außerdem die Konzentration des Reagenz genau eingestellt werden, da bei einem Überschuß eine Auflösung des Präzipitats stattfindet.

Die der vorliegenden Erfindung zugrundeliegende Aufgabe bestand darin, die Nachteile des Standes der Technik mindestens weitgehend zu vermeiden.

Diese Aufgabe wird gelöst durch Bereitstellung einer Zusammensetzung, bestehend aus mehreren verschiedenen Antikörpern oder/und Antikörperfragmenten, die spezifisch für den Fd-Abschnitt der schweren Kette von Immunglobulinen von einer oder mehreren der Klassen IgG, IgM, IgA, IgD und IgE ist und die Fähigkeit dieser Immunglobuline zur Antigenbindung zumindest weitgehend maskiert.

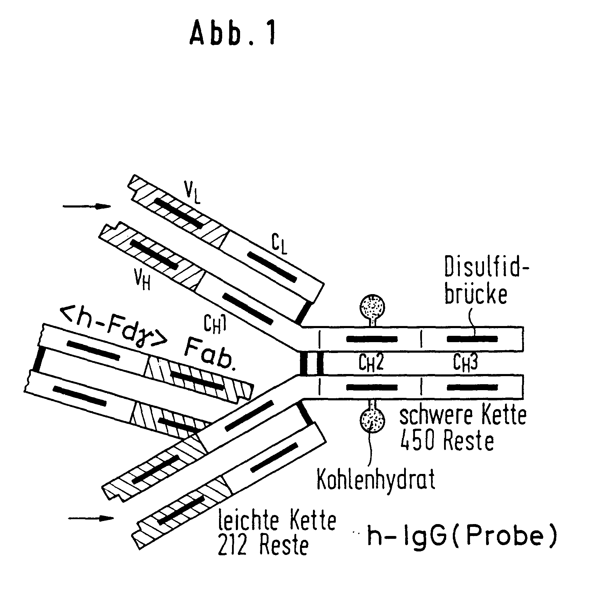

Die Bindung des Anti-Fd-Reagenz erfolgt an demjenigen Bereich der schweren Kette des zu maskierenden Immunglobulins, der zwischen der Antigenbindungsstelle und der Hinge-Region (im Bereich der V_{H} und der C_{H}¹-Domäne) liegt (vgl. Abb. 1). Vorzugsweise liegen die Bindungsstellen im konstanten Bereich der C_{H}¹-Domäne der Immunglobuline, also im C_{αH}¹-, C_{δH}¹-, C_{εH}¹-, C_{γH}¹- bzw. C_{µH}¹-Bereich (vgl. "Kurzes Lehrbuch der Immunologie", I.M. Roitt; Thieme Verlag, Stuttgart, New York (1987), Kapitel 5: "Antikörper: Struktur und Funktion", S. 49 ff.). Aminosäuresequenzen dieser Domänen sind z.B. bei Kabat et al., "Sequences of Proteins of Immunological Interest", 5th. ed. (1992), US Department of Health and Human Services, USA beschrieben.

Die Wirkung der erfindungsgemäßen Zusammensetzung besteht insbesondere darin, daß durch Vorhandensein mehrerer verschiedener Antikörper bzw. Antikörperfragmente, die gegen verschiedene Bereiche des Fd-Abschnitts einer schweren Kette gerichtet sind, die Antigenbindungsfähigkeit der betreffenden Antikörper so stark maskiert wird, so daß sie die spezifischen Bindepartner, d.h. die Antigene, nicht mehr erkennen können.

Die Zusammensetzung ist spezifisch für den Fd-Abschnitt der schweren Kette, d.h. sie ist im wesentlichen frei von Komponenten, die mit dem Fd-Abschnitt einer leichten Kette, d.h. einer *κ-* oder λ-Kette reagieren können. Die Kreuzreaktivität mit einer leichten Immunglobulinkette ist vorzugsweise maximal 10⁻¹ und besonders bevorzugt maximal 10⁻² bezüglich der Reaktivität mit der schweren Kette.

Die erfindungsgemäße Antikörper- oder/und Antikörperfragmentzusammensetzung kann in einem 5-fachen molaren Überschuß bezüglich der zu maskierenden Antikörpermoleküle deren Fähigkeit zur Antigenbindung vorzugsweise um mindestens 50 %, besonders bevorzugt um mindestens 90 % und am meisten bevorzugt um mindestens 99 % verhindern.

Die erfindungsgemäße Zusammensetzung ist vorzugsweise spezifisch für eine erste Immunglobulinklasse ausgewählt aus den Klassen IgG, IgM, IgA, IgD und IgE und weist eine Kreuzreaktivität mit einer anderen Immunglobulinklasse von maximal 10⁻², besonders bevorzugt von maximal 10⁻³ bezüglich der Reaktivität gegenüber der ersten Immunglobulinklasse auf.

In einer bevorzugten Ausführungsform der Erfindung ist die Antikörper- oder/und Antikörperfragmentzusammensetzung spezifisch für den Fd-Abschnitt der schweren Kette von Immunglobulin G, insbesondere von humanem Immunglobulin G. Diese Zusammensetzung eignet sich zur klassenspezifischen Bestimmung von Antikörpern aus einer oder mehreren der Klassen IgM, IgA, IgD und IgE in Anwesenheit von Antikörpern der Klasse IgG. Eine derartige Zusammensetzung weist vorzugsweise eine Kreuzreaktivität mit einer anderen Immunglobulinklasse, z.B. IgM, von maximal 10⁻², besonders bevorzugt von maximal 10⁻³ bezüglich der Reaktivität gegenüber IgG auf.

Eine andere bevorzugte Ausführungsform der vorliegenden Erfindung ist eine Zusammensetzung, die spezifisch für den Fd-Abschnitt der schweren Kette von Immunglobulinen der Klasse IgM ist und beispielsweise zur selektiven klassenspezifischen Bestimmung von Antikörpern der Klasse IgG verwendet werden kann.

Für einige Anwendungsformen kann es bevorzugt sein, daß die Zusammensetzung speziesspezifisch für humanes Immunglobulin ist und eine Kreuzreaktivität mit nicht-humanem Immunglobulin von maximal 10⁻², besonders bevorzugt von maximal 10⁻³ bezüglich der Reaktivität gegenüber humanem Immunglobulin aufweist.

Die erfindungsgemäße Zusammensetzung besteht aus Antikörpern oder/und Antikörperfragmenten. Vorzugsweise besteht die Zusammensetzung aus monovalenten Antikörperfragmenten, z.B. aus Fab-Fragmenten, die durch enzymatische Spaltung von Antikörpern mit Papain und anschließende Fraktionierung der Spaltprodukte erhältlich sind. Bei Verwendung einer aus monovalenten Antikörperfragmenten bestehenden Zusammensetzung wird eine Sedimentation der maskierten Antikörper vermieden.

Die erfindungsgemäße Zusammensetzung besteht aus mehreren verschiedenen Antikörpern oder/und Antikörperfragmenten, die spezifisch für jeweils unterschiedliche Bereiche des Fd-Abschnitts der schweren Immunglobulinkette sind. Vorzugsweise besteht die Zusammensetzung aus polyklonalen Antikörpern oder/und Antikörperfragmenten, sie kann jedoch auch aus einem Gemisch von mindestens zwei, vorzugsweise mindestens drei und besonders bevorzugt mindestens vier verschiedenen monoklonalen Antikörpern oder/und Antikörperfragmenten bestehen.

Eine erfindungsgemäße polyklonale Antikörper- oder/und Antikörperfragmentzusammensetzung ist erhältlich durch ein Verfahren, wobei man
(a) ein Versuchstier mit einem Immunogen, das den Fd-Abschnitt der schweren Kette von Immunglobulinen aus einer ersten Immunglobulinklasse enthält, immunisiert,
(b) ein polyklonales Antiserum aus dem Versuchstier gewinnt,
(c) das polyklonale Antiserum gegebenenfalls durch Spaltung in monovalente Antikörperfragmente überführt,
(d) das Antiserum bzw, die monovalente Antikörperfragmente einem oder mehreren Immunsorptionsschritten unterzieht, die eine Selektion auf Antikörper bzw. Antikörperfragmente erlauben, die spezifisch für den Fd-Abschnitt der schweren Kette der ersten Immunglobulinklasse sind, und
(e) eine Antikörper- bzw. Antikörperfragmentzusammensetzung mit einer Spezifität für den Fd-Abschnitt der ersten Immunglobulinklasse gewinnt.

Die Verwendung eines geeigneten Immunogens in Kombination mit der Durchführung geeigneter Immunsorptionsschritte ermöglicht die Gewinnung einer Antikörper- bzw. Antikörperfragmentzusammensetzung, die für den Fd-Abschnitt der schweren Kette einer oder mehrerer ausgewählter Immunglobulinklassen spezifisch ist und vorzugsweise im wesentlichen keine Kreuzreaktivität mit anderen Immunglobulinklassen oder speziesfremden Immunglobulinen der ausgewählten Klassen zeigt. Als Immunogene können z.B. vollständige Antikörper, Antikörperfragmente, z.B. durch enzymatische Spaltung erzeugte Antikörperfragmente wie etwa Fab, Fab' oder F(ab)'₂ oder rekombinante Antikörperfragmente, Peptidepitope oder Gemische davon verwendet werden. Die Verwendung von Immunogenen, die frei von Antikörper-Fc-Bestandteilen sind, ist bevorzugt. Auf diese Weise kann im Versuchstier, z.B. einem Schaf, einem Kaninchen oder einer Maus, die Entstehung von Anti-Fc-Antikörpern weitgehend vermieden werden.

Die Immunsorptionsschritte des erfindungsgemäßen Verfahrens umfassen vorzugsweise (i) mindestens eine positive Immunsorption gegen ein Antigen, das den Fd-Abschnitt der schweren Kette der ersten Immunglobulinklasse enthält, und die Gewinnung von bindenden Komponenten der Zusammensetzung, die spezifisch den Fd-Abschnitt der schweren Kette der ersten Immunglobulinklasse erkennen. Bei Verwendung eines Immunogens, das den Fd-Abschnitt der schweren Kette von Immunglobulin G enthält, z.B. eines Fab-Fragments, werden das Antiserum bzw. die Antikörperfragmente einer positiven Immunsorption gegen Immunglobulin G oder Fragmente davon, die den Fd-Abschnitt enthalten, unterzogen. Die Verwendung von Fc-freien Antigenen, z.B. Fab-Fragmenten, bei der positiven Immunsorption ist empfehlenswert, wenn als Immunogen ein komplettes Immunglobulin verwendet wurde.

Die Immunsorptionsschritte umfassen vorzugsweise weiterhin (ii) mindestens eine negative Immunsorption gegen Antigene, die aus Immunglobulinklassen, die von der ersten Immunglobulinklasse verschieden sind, oder Bestandteilen davon ausgewählt sind, und die Gewinnung von nicht-bindenden Komponenten der Zusammensetzung. Durch diese negative Immunsorption wird die Kreuzreaktivität der Zusammensetzung gegen den Fd-Abschnitt der leichten Kette und gegen den Fd-Abschnitt der schweren Kette anderer Immunglobulinklassen weitgehend vermindert. Zur Herstellung einer für den Fd-Abschnitt von Immunglobulin G spezifischen Zusammensetzung kann die negative Immunsorption gegen IgM, IgD, IgE oder/und IgA oder Bestandteile davon durchgeführt werden. Wenn die Absicht besteht, ein Entstörungsreagenz zur selektiven Bestimmung von IgM in Gegenwart von IgG herzustellen, führt man einen oder mehrere negative Immunsorptionsschritte gegen IgM oder Bestandteile davon durch.

Gegebenenfalls können die Immunsorptionsschritte weiterhin (iii) mindestens eine negative Immunsorption gegen Antigene, die aus der ersten Immunglobulinklasse einer Spezies, die von der Spezies der Immunglobuline im positiven Immunsorptionsschritt (i) verschieden ist, oder Bestandteilen davon ausgewählt sind, und die Gewinnung von nicht-bindenden Komponenten der Zusammensetzung umfassen. Die Durchführung dieses Schrittes ist bei einer späteren Verwendung der Zusammensetzung in einem Immunoassay bevorzugt, bei dem ein Detektionsantikörper der gleichen Immunglobulinklasse, aber aus einer anderen Spezies, verwendet wird. Vorzugsweise wird die positive Immunsorption (i) mit humanen Immunglobulinen und die negative Immunsorption (iii) mit Immunglobulinen einer nicht humanen Spezies, z.B. Maus, durchgeführt. Bei Herstellung einer für den Fd-Abschnitt von humanem Immunglobulin G spezifischen Zusammensetzung erfolgt der negative Immunsorptionsschritt (iii) gegen Immunglobulin G einer nicht humanen Spezies.

Die Durchführung der Immunsorptionsschritte erfolgt vorzugsweise durch Adsorptionschromatographie an Säulen, die die jeweils verwendeten Antigene in immobilisierter Form enthalten. Prozeduren zur Gewinnung gereinigter Antigenpräparationen und zur Immobilisierung von Antikörpern bzw. Antikörperfragmenten an ein Trägermaterial sind dem Fachmann wohlbekannt (siehe z.B. EP-A-0 394 819, insbesondere Beispiel 7).

Die erfindungsgemäße Antikörper- oder/und Antikörperfragmentzusammensetzung kann in einem Immunoassay zur selektiven klassenspezifischen Bestimmung von Antikörpern der Klassen IgG, IgM, IgA, IgD oder/und IgE verwendet werden, um die durch Antikörper anderer Klassen verursachten Störungen zu unterdrücken. Hierzu wird beispielsweise einer zu vermessenden Humanserum- oder Plasmaprobe, in der ein gegen ein bestimmtes, z.B. virales oder bakterielles Antigen gerichteter Antikörper einer bestimmten Immunglobulinklasse (z.B. IgM) klassenspezifisch quantifiziert werden soll, eine erfindungsgemäße IgG-spezifische Zusammensetzung, vorzugsweise nach einem Vorverdünnungsschritt zugesetzt, so daß das in der Probe vorhandene spezifische und unspezifische IgG im wesentlichen vollständig maskiert wird und beim anschließenden immunologischen Nachweis nur noch das IgM, nicht jedoch das IgG in der Probe erfaßt wird. Auf diese Weise ist eine differentielle Quantifizierung des spezifischen Gehalts einer bestimmten Antikörperklasse ohne Störung durch den spezifischen Gehalt einer anderen Antikörperklasse möglich. Die Verwendung der erfindungsgemäßen Zusammensetzung erlaubt eine Einschritt-Testführung ohne Waschvorgang.

Ein Gegenstand der vorliegenden Erfindung ist somit auch ein Verfahren zum selektiven immunologischen Nachweis von spezifischen Antikörpern aus einer oder mehreren ausgewählten Immunglobulinklassen in einer Probeflüssigkeit, die auch Antikörper anderer Immunglobulinklassen enthält, welches dadurch gekennzeichnet ist, daß man die Probeflüssigkeit mit einer erfindungsgemäßen Zusammensetzung vorinkubiert, um die durch Antikörper der anderen Immunglobulinklassen verursachten Störungen zu unterdrücken und dann das Vorhandensein oder/und die Menge der nachzuweisenden Antikörper aus den ausgewählten Klassen in der Probeflüssigkeit bestimmt. Die Zusammensetzung wird vorzugsweise in einer Menge zugesetzt, die einem mindestens 5-fachen molaren Überschuß der aktiven Komponenten gegenüber den in der Probeflüssigkeit vorhandenen Antikörpern der anderen Immunglobulinklassen, d.h. der nicht ausgewählten Immunglobulinklassen, entspricht. Die Zusammensetzung wird vorzugsweise in einem 10- bis 1000-fachen molaren Überschuß zugesetzt. Die Dauer der Vorinkubation beträgt vorzugsweise 5 bis 60 min und besonders bevorzugt 10 bis 30 min.

Besonders bevorzugt wird das immunologische Nachweisverfahren zur Bestimmung spezifischer Antikörper nach dem Prinzip des heterogenen Immunoassay in Gegenwart einer reaktiven Festphase und zwei mit den nachzuweisenden Antikörpern bindefähigen Rezeptoren R₁ und R₂ durchgeführt, wobei R₁ an die Festphase gebunden oder zur Bindung an die Festphase fähig ist und R₂ direkt oder indirekt markiert ist. Die nachzuweisenden Antikörper können dann gegebenenfalls nach Trennung von Festphase und Inkubationsflüssigkeit durch Messung der Markierung in der Festphase oder/und in der Inkubationsflüssigkeit bestimmt werden.

Als festphasenseitigen Rezeptor R₁ kann man ein Konjugat aus einem mit den nachzuweisenden Antikörpern spezifisch reagierenden Antigen und einer Festphasenbindungsgruppe verwenden. In diesem Falle kann der Rezeptor R₂ entweder ein mit den nachzuweisenden Antikörpern spezifisch reagierendes Antigen oder ein die ausgewählte Antikörperklasse erkennender Antikörper, z.B. ein Anti-IgM-Antikörper bzw. ein entsprechendes Antikörperfragment, sein. Der Rezeptor R₂ kann direkt oder indirekt markiert sein, d.h. er kann mit einer Markierungsgruppe gekoppelt sein oder er kann an einen weiteren Rezeptor binden, der seinerseits eine Markierungsgruppe trägt.

Als festphasenseitigen Rezeptor R₁ kann man auch ein Konjugat aus einem die ausgewählte Antikörperklasse erkennenden Antikörper bzw. einem entsprechenden Antikörperfragment und einer Festphasenbindungsgruppe verwenden. In diesem Falle verwendet man als markierten Rezeptor R₂ vorzugsweise ein mit den nachzuweisenden Antikörpern spezifisch reagierendes Antigen.

Vorzugsweise verwendet man eine mit Streptavidin oder Avidin beschichtete Festphase und einen biotinylierten Rezeptor R₁, der an diese Festphase binden kann. Als Markierung kann man chromophore Stoffe, radioaktive Isotope, Enzyme, NMR-Markierungen oder alle anderen aus dem Stand der Technik bekannten Markierungen verwenden. Vorzugsweise verwendet man lumineszierende Metallkomplexe, bei denen die Messung der Markierung durch Elektrochemilumineszenz erfolgen kann. Beispiele für lumineszierende Metallkomplexe sowie Verfahren und Vorrichtungen zur Elektrochemilumineszenzmessung sind in EP-A-0 199 804, EP-A-0 580 979, WO87/06706, WO90/05301, WO90/11511 und WO92/14138 beschrieben). Eine weitere bevorzugte Markierung sind Enzyme (z.B. Peroxidase, alkalische Phosphatase oder β-Galactosidase), bei denen die Messung der Markierung durch Nachweis der jeweiligen enzymatischen Reaktion erfolgen kann.

Noch ein weiterer Gegenstand der vorliegenden Erfindung ist ein Reagenz zum selektiven immunologischen Nachweis spezifischer Antikörper aus einer oder mehreren ausgewählten Immunglobulinklassen, das eine erfindungsgemäße Zusammensetzung enthält.

Weiterhin wird die Erfindung durch die folgenden Beispiele und Abbildungen erläutert.

Es zeigen:
- Abb. 1: die Bindung eines gegen den Fd-Abschnitt der schweren Kette gerichteten Fab-Fragments (<h-Fdγ>Fab) an humanes IgG (h-IgG),
- Abb. 2: das Testprinzip des in Beispiel 4 durchgeführten klassenspezifischen Nachweises von Anti-HBc-IgM durch Elektrochemilumineszenz und
- Abb. 3: ein weiteres Testprinzip für einen klassenspezifischen Nachweis von Anti-HBc-IgM durch Elektrochemilumineszenz.

### Beispiel 1

### Herstellung eines erfindungsgemäßen Reagenz

### 1.1 Herstellung des Immunogens

Aus Humanserum wird nach üblichen Standardverfahren die Immunglobulin G-Fraktion aufgereinigt. Diese Aufreinigung kann beispielsweise durch Aerosil-Delipidierung, Fällung mit Ammoniumsulfat, Chromatographie an DEAE-Sepharose FF und gegebenenfalls Immunsorption an immobilisierten IgG-spezifischen polyklonalen Antikörpern erfolgen.

Die so gereinigte Immunglobulinfraktion wird mit Papain in Fab- und Fc-Fragmente gespalten. Die Spaltung wird vorzugsweise bei pH 7 mit einer IgG-Konzentration von 10 mg/ml, 40 mU/ml Papain 7 und 10 mmol/l Cystein bei 37°C durchgeführt. Die Fab-Anteile werden durch DEAE-Sepharose FF-Chromatographie und Gelchromatographie (z.B. Sephacryl TSK S200 und S300) von den Fc-Anteilen gereinigt. Die Fab-Fragmente werden zur Steigerung der Immunreaktion vorzugsweise an ein Trägerprotein, z.B. Maleimidaktiviertes Keyhole Limpet-Hämocyanin (Boehringer Mannheim, Biochemica-Katalog, Best.Nr. 1376438) gekoppelt. Hierzu werden zunächst die Fab-Fragmente mit N-Succinimidyl-S-acetylthiopropionat (SATP) in einem molaren Verhältnis von 1:6 aktiviert. Die Kopplung des Trägerproteins mit dem aktivierten Fab-Fragment erfolgt in einem molaren Verhältnis von 1:1.

Auf entsprechende Weise können auch Fab-Fragmente anderer Immunglobulinklassen (z.B. Fabµ) hergestellt werden.

### 1.2 Immunisierung von Schafen

Mit dem in Beispiel 1 hergestellten Immunogen werden nach üblichen Verfahren Schafe immunisiert. Diese bilden in einer Immunreaktion polyklonale Antikörper gegen humanes Fab, die sowohl gegen den Anteil der leichten Kette als auch gegen den Anteil der schweren Kette in humanem Fab gerichtet sind.

Den immunisierten Schafen wird Rohserum entnommen und daraus die IgG-Fraktion nach dem bereits beschriebenen Verfahren gewonnen.

### 1.3 Herstellung eines Fd-spezifischen Reagenzes

Die Schaf-Immunglobulin-G-Fraktion wird mit Papain proteolytisch gespalten. Die Fab-Fragmente werden von den übrigen Spaltungsprodukten mittels DEAE-Anionenaustauscherchromatographie abgetrennt.

Aus der Gesamt-Schaf-Fab-Fraktion werden die gegen den Fd-Abschnitt der schweren Kette gerichteten Anteile durch mehrere Immunsorptionsschritte aufgereinigt.

Durch gegebenenfalls mehrfache Passage eines Immunadsorbers, auf dem humanes IgM immobilisiert ist, können die spezifisch gegen die leichte Kette (κ oder λ) des humanen IgG gerichteten Anteile entfernt werden, weil der konstante Bereich der leichten Kette von IgG und IgM homolog ist. Während die gegen die leichte Kette gerichteten Anteile auf der Säule adsorbiert werden, befinden sich die gegen den Fd-Abschnitt der schweren Kette gerichteten Anteile im Säulendurchfluß. Als Säule wird eine mit polyklonalem IgM gekoppelte Spherosilsäule verwendet. Der Auftragspuffer ist z.B. PBS/Azid (50 mmol/l K-Phosphat pH 7,5; 150 mmol/l NaCl; 0,1 % Natriumazid).

Die nicht bindenden Anteile aus der IgM-Säule werden vereinigt und einer gegebenenfalls mehrfachen Passage eines weiteren Immunadsorbers unterworfen, auf dem humanes IgG immobilisiert ist. Hier können die spezifischen Anteile an Anti-Fd-Schaf-Fab von unspezifischen Schaf-Fab-Anteilen getrennt werden. Das Fd-spezifische Schaf-Fab bindet an diese Säule und kann mit einem Elutionspuffer, z.B. 1 mol/l Propionsäure, eluiert werden. Die übrigen Komponenten binden nicht und werden durch Waschen der Säule mit einem geeigneten Puffer (z.B. PBS/Azid) entfernt.

Weiterhin können störende Kreuzreaktivitäten gegen die üblicherweise in immunologischen Tests als Bindepartner verwendeten Maus-Antikörper durch zusätzliche Passage eines Immunadsorbers mit immobilisiertem Maus-IgG entfernt: werden. Die gewünschten Anteile der Präparation sind im Säulendurchfluß.

Die Bindung eines für humanes Fdγ spezifischen Fab-Fragments (<h-Fdγ>Fab) an humanes IgG (h-IgG) ist schematisch in Abb. 1 gezeigt. Die Antigenbindungsstellen des h-IgG sind jeweils mit Pfeilen gekennzeichnet.

### Beispiel 2

### Überprüfung der Reinheit des Reagenzes

Mikrotiterplatten (MTP) der Fa. Nunc wurden mit Schaf-Fabspezifischem Kaninchen-IgG als Festphasen- oder Fängerantikörper beschichtet (10 µg/ml Kaninchen-IgG, Na-Carbonatpuffer pH 9,6, Nachbeschichtung mit 1 % Rinderserumalbumin in PBS-Puffer). Das zu analysierende Reagenz wird als Probe mit jeweils steigenden Konzentrationen (z.B. 0 bis 10 µg/ml) einer Präparation aus gereinigten humanen Fabγ-Fragmenten bzw. humanen Fabµ-Fragmenten vorinkubiert und anschließend in die beschichteten MTP überführt (Probenvolumen 200 µl, Inkubation 2 h bei Raumtemperatur, Puffer PBS mit 1 % Rinderserumalbumin).

Die für humanes Fdγ spezifischen Schaf-Fab-Komponenten der Probe werden ebenso wie gegebenenfalls vorhandene Verunreinigungen, die gegen die leichte Immunglobulinkette gerichtet sind und bei der Aufreinigung möglicherweise nicht vollständig abgetrennt wurden, an die Festphase gebunden. Nicht gebundene Anteile werden durch dreimaliges Waschen mit PBS-Puffer entfernt.

Als Nachweisreagenz wird eine an humanes Fabγ gekoppelte Meerrettich-Peroxidase eingesetzt (200 µl, 50 mU/ml). Nach dreimaligem Waschen mit PBS-Puffer wird die enzymatische Aktivität des gebundenen Peroxidasekonjugats durch einstündige Inkubation bei Raumtemperatur mit dem Substrat ABTS® entwickkelt und anschließend photometrisch bei 405 nm vermessen. Von den zuvor an die Festphase gebundenen Schaf-Fab-Komponenten der Probe werden vom Konjugat-Enzymmarker nur die gegen humanes Fab gerichteten Anteile erkannt. Dadurch wird in der Kontrolle ohne Fabµ- und Fabγ-Zusatz ein Positivsignal von 1000 bis 3000 mE erzeugt. Dieses entspricht der Gesamtmenge des in einer zu untersuchenden Präparation vorhandenen, gegen humanes Fab gerichteten Schaf-Fab. In den Proben, denen steigende Menge an humanem Fabγ zugesetzt wurden, kommt es durch Maskierung des Fdγ-spezifischen Schaf-Fab zu einer zunehmenden Verdrängung des Konjugats und somit zu einer gegen 0- bzw. gegen einen Leerwert strebenden Kurve des Meßsignals. In den Proben, denen steigende Mengen an humanem Fabµ beigemischt wurden, können dagegen nur die gegen die leichte Kette gerichteten Schaf-Fab-Anteile maskiert werden, so daß es hier nur zu einem Signalabfall kommt, wenn solche unerwünschten Komponenten noch in der Schaf-Fab-Präparation verhanden sind. Die Differenz der beiden Verdrängungskurven würde in letzterem Fall dem tatsächlichen Anteil an Fdγ-spezifischen Schaf-Fab in der Präparation entsprechen.

Die Messung der Probe ohne zugesetztes humanes Fabµ bzw. Fabγ ergibt eine Extinktion von 1600 mE. Die Probe + Fabµ ergibt eine Extinktion von 1500 mE. Die Probe + Fabγ ergibt eine Extinktion von 150 mE.

### Beispiel 3

### Verwendung eines Fd-spezifischen Entstörungsreagenzes in einem Enzymuntest

Um die Funktionsfähigkeit der in Beispiel 1 hergestellten Anti-Fdγ-Antikörperfragmentpräparation zu zeigen, wurde Anti-Fdγ einer Humanserumprobe zugesetzt, die gegen das Core-Antigen von Hepatitis B-Virus (HBc-Antigen) gerichtete Antikörper (<HBc>) enthält. Anschließend wurde HBc-Antigen zugegeben und inkubiert. Das Anti-HBc-spezifische IgG kann nun, soweit es nicht von Anti-Fdγ markiert worden ist, mit diesem Antigen reagieren. Anschließend wurde überschüssiges IgG (spezifisch für HBc-Antigen und unspezifisch) durch Waschen abgetrennt. Das an HBc-Antigen gebundene IgG kann nun durch ein Peroxidase-markiertes Anti-IgG, welches an den Fcγ-Teil von IgG bindet, nachgewiesen werden.

Die Durchführung des Tests war wie folgt:

Die Anti-HBc-IgG-haltigen Proben wurden mit verschiedenen Mengen an Anti-Fdγ versetzt und 30 min bei Raumtemperatur inkubiert. Anschließend wurden die Proben mit Phosphatpuffer 40 mmol/l, pH 7,4 auf 1:100 weiterverdünnt.

In einem Streptavidin-beschichteten Testgefäß wurden 20 *µ* l der verdünnten Probe zu 500 µl einer Lösung von biotinyliertem HBc-Antigen (10 µg/ml aus dem Enzymuntest® Anti-HBc) gegeben und 10 min inkubiert. Anschließend wurden 500 µl eines Konjugats aus Peroxidase und Anti-IgG-Antikörper (50 mU/ml aus dem Enzymuntest® Anti-HCV) zugegeben, 60 min inkubiert und gewaschen. Anschließend wurden 1000 µl Substratlösung (H₂O₂ (Natriumperborat) 3,2 mmol/l aus dem Enzymuntest® Anti-HBc) zugegeben und 60 min inkubiert. Schließlich wurde die Extinktion am Analysengerät ES600 (Boehringer Mannheim GmbH) bestimmt.

In Tabelle 1 sind die Ergebnisse dieses Versuchs dargestellt. Es ist klar zu erkennen, daß die erfindungsgemäße Anti-Fdγ-Zusammensetzung die Fähigkeit zur Antigenbindung des IgG verändert, so daß es nicht mehr binden kann.

**Tabelle 1:**

| Probe | Gehalt<HBc> [U/ml] | Extinktion (E) bei Zusatz von anti-Fdγ zur unverdünnten Probe [mg/ml] | | | | |
|---|---|---|---|---|---|---|
| | | 0 | 10 | 50 | 100 | 200 |
| 0 | 0 | 0,07 | 0,06 | 0,05 | 0,04 | 0,03 |
| 1 | 22000 | 1,63 | 1,35 | 0,63 | 0,4 | 0,16 |
| 2 | 6000 | 0,91 | 0,82 | 0,56 | 0,25 | 0,07 |
| 3 | 12000 | 1,05 | 1,0 | 0,69 | 0,25 | 0,07 |

### Beispiel 4

### Klassenspezifischer Nachweis von Anti-HBc-IgM durch Elektrochemilumineszenz

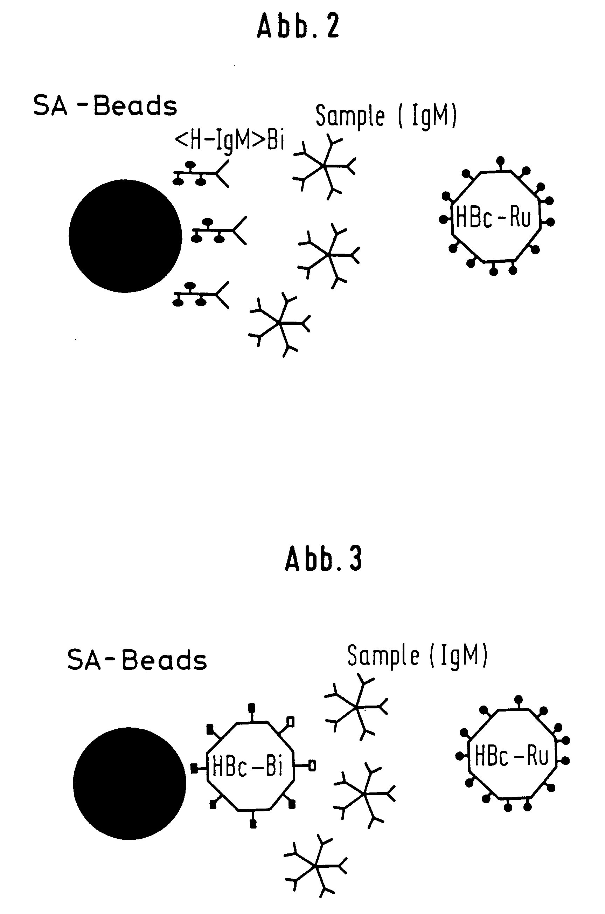

Zur Messung wurde ein Apparat verwendet, wie er in WO90/05302 beschrieben ist. Das Testprinzip ist in Abb. 2 dargestellt und beruht auf der Verwendung eines biotinylierten, gegen humanes IgM spezifischen Antikörpers (<H-IGM>Bi) und eines direkt mit einem Rutheniumkomplex markierten HBc-Antigens (HBc-Ru). Alternativ kann auch ein indirekt (durch Bindung eines ruthenylierten Anti-HBc-Antikörpers) markiertes HBc-Antigens eingesetzt werden. Der Rutheniumkomplex und die zur Kopplung an das HBc-Antigen bzw. an den Antikörper verwendeten Techniken sind in EP-A-0 199 804 beschrieben.

Ein weiteres Testprinzip ist in Abb.3 gezeigt. Es beruht auf der Verwendung eines biotinylierten HBc-Antigens (HBc-Bi) und eines Ruthenium-markierten HBc-Antigens (HBc-Ru).

Die Durchführung war wie folgt:
90 µl einer Lösung von unspezifischem humanem IgM (11 µg/ml) mit bzw. ohne 1000 µg/ml Anti-Fdγ wurden mit 10 µl von 1:100 vorverdünnten Proben bzw. Standards versetzt und 9 min bei 37°C inkubiert.

Anschließend wurden 100 µl einer Lösung, die Ruthenium-markiertes HBc-Antigen und einen biotinylierten Anti-human-IgM-Antikörper (IgG) enthielt, sowie 50 µl Streptavidin beschichtete Magnetpartikel (Fa. Dynal) zugesetzt, das Gemisch danach weitere 9 min bei 37°C inkubiert, anschließend mit Assaypuffer (200 mmol/l Phosphat, pH 6,8, Polydocanol 0,1 %, Oxaban A 0,1 %, Tripropylamin 160 mmol/l) in die auf 28°C temperierte Meßzelle überführt und dort vermessen.

Die IgM-Konzentration in den Proben wurde anschließend anhand einer Eichkurve bestimmt. Die Ergebnisse auf Konzentrationsbasis sind in der Tabelle 2 dargestellt. Es ist zu erkennen, daß spezifisches Anti-HBc-IgG bei einem Elektrochemilumineszenznachweisverfahren an das HBc-Antigen binden kann, so daß eine zu niedrige Konzentration an Anti-HBc-IgM gemessen wird. Durch Zusatz von Anti-Fdγ wird die Bindefähigkeit von spezifischem IgG an das HBc-Antigen, aber auch die Bindefähigkeit von unspezifischem IgG an andere Antigene maskiert.

Wie aus der Tabelle ersichtlich ist, erhöhen sich die in den Proben gemessenen IgM-Konzentrationen durch Zugabe von Anti-Fdγ, so daß der im Enzymunsystem (wo aufgrund eines Waschvorgangs keine IgG-Störung auftritt) gemessene IgM-Konzentrationsbereich getroffen wird.

Anmerkung: Die Proben 1 bis 4 enthielten 6000, 12000, 6900 bzw. 4500 U/ml HBc-spezifisches IgG, d.h. einen erheblichen Überschuß. Durch Zugabe von Fdγ wird die durch IgG verursachte Störung beseitigt.

**Tabelle 2**

| Probe | Enzymun <HBc>IgM [U/ml] | Flash <HBc>IgM ohne <Fdγ> [U/ml] | Flash <HBc>IgM mit <Fdγ> [U/ml] |
|---|---|---|---|
| 1 | 249 | 116 | 281 |
| 2 | 56 | 21 | 51 |
| 3 | 62 | 20 | 96 |
| 4 | 140 | 39 | 110 |

## Patentansprüche

1. Zusammensetzung, bestehend aus mehreren verschiedenen Antikörpern oder/und Antikörperfragmenten, die spezifisch für den Fd-Abschnitt der schweren Kette von Immunglobulinen aus einer oder mehreren der Klassen IgG, IgM, IgA, IgD und IgE ist und die Fähigkeit dieser Immunglobuline zur Antigenbindung zumindest weitgehend maskiert, erhältlich durch ein Verfahren, wobei man
(a) ein Versuchstier mit einem Immunogen, das den Fd-Abschnitt der schweren Kette von Immunglobulinen aus einer ersten Immunglobulinklasse enthält, immunisiert,
(b) ein polyklonales Antiserum aus dem Versuchstier gewinnt,
(c) das polyklonale Antiserum gegebenenfalls durch Spaltung in monovalente Antikörperfragmente überführt,
(d) das Antiserum bzw. die monovalente Antikörperfragmente einem oder mehreren Immunsorptionsschritten unterzieht, die eine Selektion auf Antikörper bzw. Antikörperfragmente erlauben, die spezifisch für den Fd-Abschnitt der schweren Kette der ersten Immunglobulinklasse sind, wobei die Immunsorptionsschritte umfassen:
(i) mindestens eine positive Immunsorption gegen ein Antigen, das den Fd-Abschnitt der schweren Kette der ersten Immunglobulinklasse enthält, und die Gewinnung von bindenden Komponenten der Zusammensetzung und
(ii) mindestens eine negative Immunsorption gegen Antigene, die aus Immunglobulinklassen, die von der ersten Immunglobulinklasse verschieden sind, oder Bestandteilen davon ausgewählt sind, und die Gewinnung von nicht bindenden Komponenten der Zusammensetzung,und
(e) eine Antikörper- bzw. Antikörperfragmentzusammensetzung mit einer Spezifität für den Fd-Abschnitt der ersten Immunglobulinklasse gewinnt.

2. Zusammensetzung nach Anspruch 1,
**dadurch gekennzeichnet,**
daß sie spezifisch für den Fd-Abschnitt der schweren Kette von Immunglobulin G ist.

3. Zusammensetzung nach Anspruch 1 oder 2,
**dadurch gekennzeichnet,**
daß sie in einem 5-fachen molaren Überschuß bezüglich der zu maskierenden Antikörpermoleküle deren Fähigkeit zur Antigenbindung um mindestens 50 % verhindert.

4. Zusammensetzung nach einem der vorhergehenden Ansprüche,
**dadurch gekennzeichnet,**
daß sie spezifisch für eine erste Immunglobulinklasse ist und eine Kreuzreaktivität mit einer anderen Immunglobulinklasse von maximal 10⁻² bezüglich der Reaktivität gegenüber der ersten Immunglobulinklasse aufweist.

5. Zusammensetzung nach Anspruch 4,
**dadurch gekennzeichnet,**
daß sie spezifisch für IgG ist und eine Kreuzreaktivität mit IgM von maximal 10⁻² bezüglich der Reaktivität gegenüber IgG aufweist.

6. Zusammensetzung nach einem der vorhergehenden Ansprüche,
**dadurch gekennzeichnet,**
daß sie spezifisch für humanes Immunglobulin ist und eine Kreuzreaktivität mit nicht-humanem Immunglobulin von maximal 10⁻² bezüglich der Reaktivität gegenüber humanem Immunglobulin aufweist.

7. Verfahren zur Herstellung einer Zusammensetzung nach einem der vorhergehenden Ansprüche,
**dadurch gekennzeichnet,**
daß man
(a) ein Versuchstier mit einem Immunogen, das den Fd-Abschnitt der schweren Kette von Immunglobulinen aus einer ersten Immunglobulinklasse enthält, immunisiert,
(b) ein polyklonales Antiserum aus dem Versuchstier gewinnt,
(c) das polyklonale Antiserum gegebenenfalls durch Spaltung in monovalente Antikörperfragmente überführt,
(d) das Antiserum bzw. die monovalente Antikörperfragmente einem oder mehreren Immunsorptionsschritten unterzieht, die eine Selektion auf Antikörper bzw. Antikörperfragmente erlauben, die spezifisch für den Fd-Abschnitt der schweren Kette der ersten Immunglobulinklasse sind, wobei die Immunsorptionsschritte umfassen:
(i) mindestens eine positive Immunsorption gegen ein Antigen, das den Fd-Abschnitt der schweren Kette der ersten Immunglobulinklasse enthält, und die Gewinnung von bindenden Komponenten der Zusammensetzung und
(ii) mindestens eine negative Immunsorption gegen Antigene, die aus Immunglobulinklassen, die von der ersten Immunglobulinklasse verschieden sind, oder Bestandteilen davon ausgewählt sind, und die Gewinnung von nicht bindenden Komponenten der Zusammensetzung, und
(e) eine Antikörper- bzw. Antikörperfragmentzusammensetzung mit einer Spezifität für den Fd-Abschnitt der ersten Immunglobulinklasse gewinnt.

8. Verfahren nach Anspruch 7,
**dadurch gekennzeichnet,**
daß man ein Immunogen verwendet, das keine Fc-Bestandteile von Immunglobulinen enthält.

9. Verfahren nach Anspruch 1 oder 7,
**dadurch gekennzeichnet,**
daß die Immunsorptionsschritte weiterhin (iii) mindestens eine negative Immunsorption gegen Antigene, die aus der ersten Immunglobulinklasse einer Spezies, die von der Spezies der Immunglobuline im positiven Immunsorptionsschritt (i) verschieden ist, oder Bestandteilen davon ausgewählt sind, und die Gewinnung von nicht bindenden Komponenten der Zusammensetzung umfassen.

10. Verfahren nach Anspruch 9,
**dadurch gekennzeichnet,**
daß man die positive Immunsorption (i) mit humanen Immunglobulinen der ersten Immunglublinklasse und die negative Immunsorption (iii) mit Immunglobulinen einer nicht-humanen Spezies der ersten Immunglobulinklasse durchführt.

11. Verfahren nach einem der Ansprüche 7 bis 10,
**dadurch gekennzeichnet,**
daß man das polyklonale Antiserum durch Spaltung mit Papain in Fab-Fragmente überführt.

12. Verwendung einer Zusammensetzung nach einem der Ansprüche 1-6, in einem Immunoassay zur selektiven klassenspezifischen Bestimmung von Antikörpern der Klassen IgG, IgM, IgA, IgD oder/und IgE, um die durch Antikörper anderer Klassen verursachten Störungen zu unterdrücken.

13. Verfahren zum selektiven immunologischen Nachweis von spezifischen Antikörpern aus einer oder mehreren ausgewählten Immunglobulinklassen in einer Probeflüssigkeit, die auch Antikörper anderer Immunglobulinklassen enthält,
**dadurch gekennzeichnet,**
daß man die Probeflüssigkeit mit einer Zusammensetzung nach einem der Ansprüche 1-6 vorinkubiert, um die durch Antikörper der anderen Immunglobulinklassen verursachten Störungen zu unterdrücken und dann das Vorhandensein oder/und die Menge der nachzuweisenden Antikörper aus den ausgewählten Klassen in der Probeflüssigkeit bestimmt.

14. Verfahren nach Anspruch 13,
**dadurch gekennzeichnet,**
daß man die Zusammensetzung in einer Menge zusetzt, die einem mindestens 5-fachen molaren Überschuß der aktiven Komponenten gegenüber den in der Probeflüssigkeit vorhandenen Antikörpern der anderen Immunglobulinklassen entspricht.

15. Verfahren nach einem der Ansprüche 13 bis 14,
**dadurch gekennzeichnet,**
daß die Bestimmung nach dem Prinzip des heterogenen Immunoassay in Gegenwart einer reaktiven Festphase und zwei mit den nachzuweisenden Antikörpern bindefähigen Rezeptoren R₁ und R₂ erfolgt, wobei R₁ an die Festphase gebunden oder zur Bindung an die Festphase fähig ist und R₂ direkt oder indirekt markiert ist und die nachzuweisenden Antikörper durch Messung der Markierung in der Festphase oder/und in der Inkubationsflüssigkeit bestimmt werden.

16. Reagenz zum selektiven immunologischen Nachweis spezifischer Antikörper aus einer oder mehreren ausgewählten Immunglobulinklassen ,
**dadurch gekennzeichnet,**
daß es eine Zusammensetzung nach einem der Ansprüche 1 bis 6 enthält.

## Claims

1. Composition composed of several different antibodies or/and antibody fragments which is specific for the Fd section of the heavy chain of immunoglobulins from one or several of the classes IgG, IgM, IgA, IgD and IgE and which at least substantially masks the ability of these immunoglobulins to bind antigens obtainable by a process in which
(a) an experimental animal is immunized with an immunogen which contains the Fd section of the heavy chain of immunoglobulins from a first immunoglobulin class,
(b) a polyclonal antiserum is obtained from the experimental animal,
(c) the polyclonal antiserum is optionally converted by cleavage into monovalent antibody fragments,
(d) the antiserum or the monovalent antibody fragments are subjected to one or several immunosorption steps which allow a selection for antibodies or/and antibody fragments which are specific for the Fd section of the heavy chain of the first immunoglobulin class and the immunosorption steps comprise
(i) at least one positive immunosorption against an antigen which contains the Fd section of the heavy chain of the first immunoglobulin class and the isolation of binding components of the composition and
(ii) at least one negative immunosorption against antigens which are selected from immunoglobulin classes which are different from the first immunoglobulin class, or components thereof, and the isolation of non-binding components of the composition and
(e) an antibody or antibody fragment composition with a specificity for the Fd section of the first immunoglobulin class is isolated.

2. Composition as claimed in claim 1,
**wherein**
it is specific for the Fd section of the heavy chain of immunoglobulin G.

3. Composition as claimed in claim 1 or 2,
**wherein**
in a 5-fold molar excess relative to the antibody molecules to be masked it inhibits their ability to bind antigens by at least 50 %.

4. Composition as claimed in one of the previous claims,
**wherein**
it is specific for a first immunoglobulin class and has a maximum cross-reactivity with another immunoglobulin class of 10⁻² relative to the reactivity towards the first immunoglobulin class.

5. Composition as claimed in claim 4,
**wherein**
it is specific for IgG and has a maximum cross-reactivity with IgM of 10⁻² relative to the reactivity towards IgG.

6. Composition as claimed in one of the previous claims,
**wherein**
it is specific for human immunoglobulin and has a maximum cross-reactivity with non-human immunoglobulin of 10⁻² relative to the reactivity towards human immunoglobulin.

7. Process for the production of a composition as claimed in one of the previous claims,
**wherein**
(a) an experimental animal is immunized with an immunogen which contains the Fd section of the heavy chain of immunoglobulins from a first immunoglobulin class,
(b) a polyclonal antiserum is obtained from the experimental animal,
(c) the polyclonal antiserum is optionally converted by cleavage into monovalent antibody fragments,
(d) the antiserum or the monovalent antibody fragments are subjected to one or several immunosorption steps which allow a selection for antibodies or/and antibody fragments which are specific for the Fd section of the heavy chain of the first immunoglobulin class and the immunosorption steps comprise
(i) at least one positive immunosorption against an antigen which contains the Fd section of the heavy chain of the first immunoglobulin class and the isolation of binding components of the composition and
(ii) at least one negative immunosorption against antigens which are selected from immunoglobulin classes which are different from the first immunoglobulin class, or components thereof, and the isolation of non-binding components of the composition and
(e) an antibody or antibody fragment composition with a specificity for the Fd section of the first immunoglobulin class is isolated.

8. Process as claimed in claim 7,
**wherein**
an immunogen is used which contains no Fc components of immunoglobulins.

9. Process as claimed in claim 1 or 7,
**wherein**
the immunosorption steps furthermore comprise (iii) at least one negative immunosorption against antigens which are selected from the first immunoglobulin class of a species which is different from the species of the immunoglobulins in the positive immunosorption step (i), or components thereof, and the isolation of nonbinding components of the composition.

10. Process as claimed in claim 9,
**wherein**
the positive immunosorption (i) is carried out with human immunoglobulins of the first immunoglobulin class and the negative immunosorption (iii) is carried out with immunoglobulins of a non-human species of the first immunoglobulin class.

11. Process as claimed in one of the claims 7 to 10,
**wherein**
the polyclonal antiserum is converted into Fab fragments by cleavage with papain.

12. Use of a composition as claimed in one of the claims 1-6 in an immunoassay for the selective class-specific determination of antibodies of the classes IgG, IgM, IgA, IgD or/and IgE in order to suppress interferences caused by antibodies of other classes.

13. Method for the selective immunological detection of specific antibodies from one or several selected immunoglobulin classes in a sample liquid which also contains antibodies of other immunoglobulin classes,
**wherein**
the sample liquid is pre-incubated with a composition as claimed in one of the claims 1 - 6 in order to suppress interferences caused by the other immunoglobulin classes and then the presence or/and amount of the antibodies of the selected classes to be detected is determined in the sample liquid.

14. Method as claimed in claim 13,
**wherein**
the composition is added in an amount which corresponds to an at least 5-fold molar excess of the active components relative to the antibodies of the other immunoglobulin classes present in the sample liquid.

15. Method as claimed in one of the claims 13 to 14,
**wherein**
the determination is carried out according to the principle of a heterogeneous immunoassay in the presence of a reactive solid phase and two receptors R₁ and R₂ capable of binding to the antibodies to be detected, in which R₁ is bound to the solid phase or is capable of binding to the solid phase and R₂ is directly or indirectly labelled and the antibodies to be detected are determined by measuring the label in the solid phase or/and in the incubation liquid.

16. Reagent for the selective immunological detection of specific antibodies from one or several selected immunoglobulin classes,
**wherein**
it contains a composition as claimed in one of the claims 1 to 6.

## Revendications

1. Composition consistant en plusieurs anticorps et/ou fragments d'anticorps différents qui est spécifique du segment Fd de la chaîne lourde d'immunoglobulines d'une ou plusieurs des classes IgG, IgM, IgA, IgD et IgE et qui masque au moins largement l'aptitude de ces immunoglobulines à lier des antigènes, pouvant être obtenue par un procédé où
(a) on immunise un animal d'expérience avec un immunogène qui contient le segment Fd de la chaîne lourde d'immunoglobulines d'une première classe d'immunoglobulines,
(b) on obtient un antisérum polyclonal à partir de l'animal d'expérience,
(c) on convertit éventuellement par clivage en fragments d'anticorps monovalents l'antisérum polyclonal,
(d) on soumet l'antisérum ou les fragments d'anticorps monovalents à une ou plusieurs étapes d'immunosorption qui permettent une sélection d'anticorps ou de fragments d'anticorps qui sont spécifiques du segment Fd de la chaîne lourde de la première classe d'immunoglobulines, où les étapes d'immunosorption comprennent :
(i) au moins une immunosorption positive contre un antigène qui contient le segment Fd de la chaîne lourde de la première classe d'immunoglobulines, et l'obtention de composants liants de la composition et
(ii) au moins une immunosorption négative contre des antigènes qui sont choisis parmi des classes d'immunoglobulines qui sont différentes de la première classe d'immunoglobulines, ou des constituants de celles-ci, et l'obtention de composants non liants de la composition, et
(e) on obtient une composition d'anticorps ou de fragments d'anticorps ayant une spécificité pour le segment Fd de la première classe d'immunoglobulines.

2. Composition selon la revendication 1 caractérisée en ce qu'elle est spécifique du segment Fd de la chaîne lourde d'immunoglobuline G.

3. Composition selon la revendication 1 ou 2 caractérisée en ce que, en un excès molaire de 5 fois par rapport aux molécules d'anticorps à masquer, elle empêche leur aptitude à lier des antigènes à raison d'au moins 50 %.

4. Composition selon l'une des revendications précédentes caractérisée en ce qu'elle est spécifique d'une première classe d'immunoglobulines et présente une réactivité croisée avec une autre classe d'immunoglobulines d'au maximum 10⁻² par rapport à la réactivité à l'égard de la première classe d'immunoglobulines.

5. Composition selon la revendication 4 caractérisée en ce qu'elle est spécifique d'IgG et présente une réactivité croisée avec IgM d'au maximum 10⁻² par rapport à la réactivité à l'égard d'IgG.

6. Composition selon l'une des revendications précédentes caractérisée en ce qu'elle est spécifique des immunoglobulines humaines et présente une réactivité croisée avec les immunoglobulines non humaines d'au maximum 10⁻² par rapport à la réactivité à l'égard des immunoglobulines humaines.

7. Procédé de préparation d'une composition selon l'une des revendications précédentes caractérisé en ce que
(a) on immunise un animal d'expérience avec un immunogène qui contient le segment Fd de la chaîne lourde d'immunoglobulines d'une première classe d'immunoglobulines,
(b) on obtient un antisérum polyclonal à partir de l'animal d'expérience,
(c) on convertit éventuellement par clivage en fragments d'anticorps monovalents l'antisérum polyclonal,
(d) on soumet l'antisérum ou les fragments d'anticorps monovalents à une ou plusieurs étapes d'immunosorption qui permettent une sélection d'anticorps ou de fragments d'anticorps qui sont spécifiques du segment Fd de la chaîne lourde de la première classe d'immunoglobulines, où les étapes d'immunosorption comprennent :
(i) au moins une immunosorption positive contre un antigène qui contient le segment Fd de la chaîne lourde de la première classe d'immunoglobulines, et l'obtention de composants liants de la composition et
(ii) au moins une immunosorption négative contre des antigènes qui sont choisis parmi des classes d'immunoglobulines qui sont différentes de la première classe d'immunoglobulines, ou des constituants de celles-ci, et l'obtention de composants non liants de la composition, et
(e) on obtient une composition d'anticorps ou de fragments d'anticorps ayant une spécificité pour le segment Fd de la première classe d'immunoglobulines.

8. Procédé selon la revendication 7 caractérisé en ce que l'on utilise un immunogène qui ne contient aucun constituant Fc d'immunoglobulines.

9. Procédé selon la revendication 1 ou 7 caractérisé en ce que les étapes d'immunosorption comprennent en outre (iii) au moins une immunosorption négative contre des antigènes qui sont choisis parmi la première classe d'immunoglobulines d'une espèce qui est différente de l'espèce des immunoglobulines dans l'étape d'immunosorption positive (i), ou des constituants de celle-ci, et l'obtention de composants non liants de la composition.

10. Procédé selon la revendication 9 caractérisé en ce que l'on réalise l'immunosorption positive (i) avec des immunoglobulines humaines de la première classe d'immunoglobulines et l'immunosorption négative (iii) avec des immunoglobulines d'une espèce non humaine de la première classe d'immunoglobulines.

11. Procédé selon l'une des revendications 7 à 10 caractérisé en ce que l'on convertit l'antisérum polyclonal par clivage avec la papaïne en fragments Fab.

12. Utilisation d'une composition selon l'une des revendications 1-6 dans un immunodosage pour la détermination spécifique de classe sélective d'anticorps des classes IgG, IgM, IgA, IgD et/ou IgE pour réprimer les perturbations provoquées par des anticorps d'autres classes.

13. Procédé pour la mise en évidence immunologique sélective d'anticorps spécifiques issus d'une ou plusieurs classes d'immunoglobulines choisies dans un liquide échantillon qui contient aussi des anticorps d'autres classes d'immunoglobulines caractérisé en ce que l'on préincube le liquide échantillon avec une composition selon l'une des revendications 1-6 pour réprimer les perturbations provoquées par des anticorps des autres classes d'immunoglobulines puis on détermine dans le liquide échantillon la présence et/ou la quantité des anticorps à mettre en évidence issus des classes choisies.

14. Procédé selon la revendication 13 caractérisé en ce que l'on ajoute la composition en une quantité qui correspond à un excès molaire d'au moins 5 fois des composants actifs par rapport aux anticorps des autres classes d'immunoglobulines présents dans le liquide échantillon.

15. Procédé selon l'une des revendications 13 à 14 caractérisé en ce que la détermination a lieu selon le principe de l'immunodosage hétérogène en présence d'une phase solide réactive et de deux récepteurs R₁ et R₂ capables de se lier aux anticorps à mettre en évidence, où R₁ est lié à la phase solide ou est capable de se lier à la phase solide et R₂ est marqué directement ou indirectement et les anticorps à mettre en évidence sont déterminés par mesure du marqueur dans la phase solide et/ou dans le liquide d'incubation.

16. Réactif pour la mise en évidence immunologique sélective d'anticorps spécifiques issus d'une ou plusieurs classes d'immunoglobulines choisies caractérisé en ce qu'il contient une composition selon l'une des revendications 1 à 6.
